# EUROPÄISCHE PATENTANMELDUNG

(11) **EP 1 580 220 A1**
(43) Veröffentlichungstag der Anmeldung: **28.09.2005**
(21) Anmeldenummer: 05005354.5
(22) Anmeldetag: 11.03.2005
(51) Int. Cl.: C08J 9/06

(54) **Feste Treibmittelpräparationen und Verfahren zu ihrer Herstellung**

(30) Priorität: 20.03.2004 DE 102004013797
(71) Anmelder: LANXESS Deutschland GmbH, 51369 Leverkusen (DE)
(72) Erfinder: Nyssen, Peter-Roger, 41542 Dormagen (DE)

(57) **Zusammenfassung**

Die vorliegende Erfindung betrifft neuartige Treibmittelpräparationen, ein Verfahren zu ihrer Herstellung sowie deren Verwendung. Die vorliegende Erfindung betrifft auch ein Verfahren zur Herstellung von Azodicarbonamid.

## Beschreibung

Die vorliegende Erfindung betrifft neuartige Treibmittelpräparationen, ein Verfahren zu ihrer Herstellung sowie deren Verwendung. Die vorliegende Erfindung betrifft auch ein Verfahren zur Herstellung von Azodicarbonamid.

Treibmittel werden technisch u.a. zur Verschäumung von PVC, Gummi, Polyolefinen wie Polyethylen oder Polypropylen sowie anderen thermoplastischen Polymeren verwendet. Die chemische Synthese von Azodicarbonamid als eines der bedeutendsten Treibmittel ist allgemein bekannt und kann beispielsweise aus der DE 69 116 867 A1 entnommen werden. Heute setzt man diese Treibmittel in Form ihrer feinteiligen Pulver ein, in geringerem Umfang auch Treibmittelzubereitungen als Mischungen mit Aktivatoren und/oder anderen Treibmitteln sowie als polymer-spezifische Masterbatche. Je nach gewünschter Anwendung besitzen die Treibmittelpulver unterschiedliche Partikelfeinheiten, die nach an sich bekannten Verfahren der Luftstrahlmahlung, wie z.B. Mahlung auf Spiralstrahlmühlen, im Anschluss an die chemische Synthese und Trocknung hergestellt werden. Die so hergestellten Pulver haben mittlere Primärpartikelgrößen (massebezogen) von 2 bis 100 *µ*m, breite Partikelgrößenverteilungen und führen deshalb zu hohen Staubbelastungen bei der Herstellung und in den Anwendungen. Stäube von Treibmittelpulvern sind im Allgemeinen zudem staubexplosionsfähig und/oder deflagrationsfähig. Ein weiterer Nachteil der bekannten Treibmittelpulver ist das schlechte Fließverhalten bedingt durch die Pulvermorphologie und die breite Partikelverteilung mit hohem Feinanteil der Primärpartikel. Dies gilt insbesondere für Azodicarbonamid.

Zur Verbesserung des Staubverhaltens werden in EP 0 943 655 A1, ähnlich wie bei Farbstoffen und Pigmenten bereits lange bekannt, ölähnliche Substanzen wie z.B. natürliche Fette und Öle, langkettige Kohlenwasserstoffe und Fettsäuren als Staubbindemittel auf die Pulver aufgebracht und eingemischt. Hierdurch kann jedoch die Staubbildung nur teilweise verbessert werden, insbesondere fuhrt ein hoher Gehalt an Staubbindemittel bekanntlich zu schlechterem Fließverhalten bzw. Verklebung und/oder Verklumpung der Treibmittel. Die Lagerfähigkeit der Produkte wird dadurch verschlechtert.

Polymer Masterbatches in Form einer Mischung aus Treibmittel und spezifischen Polymeren haben im Allgemeinen zwar Granulatform und ein besseres Staubverhalten als die reinen Treibmittelpulver, leider sind sie aufgrund der verwendeten speziellen Polymeren nicht universell verwendbar.

In JP 3438043 werden staubarme Treibmittelgranulate beschrieben, die neben dem Treibmittel ein oberflächenaktives Mittel (surfactant) und/oder ein organisches oder anorganisches Bindemittel enthalten. Die Granulate werden durch Aufbauagglomeration in einem Mischer und/oder Fließbett erhalten, indem Bindemittel und oberflächenaktives Mittel als wässrige Formulierung dem Treibmittel zugesetzt und getrocknet werden. Durch die geeignete Wahl des oberflächenaktiven Mittels wird eine verbesserte Dispergierbarkeit im Anwendungsmedium erwartet. Die Verwendung eines Bindemittels ist verfahrensbedingt notwendig (es bindet die Treibmittel-Primärpartikel in den Granulaten), im Hinblick auf die anwendungsgemäße Redispergierbarkeit und universelle Verwendbarkeit der Granulate jedoch nachteilig. Ferner sind verfahrensbedingte Nachteile zu erwarten, so z.B. Aggregation von Primärpartikeln infolge inhomogener Belegung mit den genannten Mitteln oder unerwünschte Veränderung der Kornverteilung der Treibmittel-Primärpartikel durch den Energieeintrag während des nachträglichen Mischprozesses.

Wie oben bereits erwähnt, werden Treibmittel, wie z.B. Azodicarbonamid im Anschluss an ihre Synthese und Trocknung mittels Verfahren der Trockenmahlung auf die gewünschte Feinverteilung der Primärpartikel vermahlen. Aufgrund der Explosionsfähigkeit der Produkte werden vorzugsweise Luftstrahlmühlen eingesetzt, wie z.B. Spiralstrahlmühlen, deren Nachteile in einem hohen spezifischen Energieeintrag - gleichbedeutend mit hohen Mahlkosten - und einer breiten Partikelgrößenverteilung der erhaltenen Produkte liegen. Mittlere Primärpartikeldurchmesser unter 2 *µ*m sind mit Luftstrahlmühlen unter technisch vertretbarem Energieaufwand nicht erreichbar. Für die Zerkleinerung z.B. von Azodicarbonamid mit einer mittleren Ausgangspartikelgröße von ca. 25 *µ*m auf mittlere Primärpartikelgrößen von 4 bis 2 *µ*m beträgt der spezifische Energieeintrag bei Mahlung mit Luft auf Spiralstrahlmühlen etwa 6000 bis 12000 kJ/kg Produkt.

Der Erfindung lag die Aufgabe zugrunde, staubarme Treibmittelpräparationen bereitzustellen, die ohne Agglomeration und ohne Zuhilfenahme eines Bindemittels hergestellt werden, die eine engere Primärpartikelverteilung aufweisen und die umweltfreundlich in ihrer Herstellung sind aufgrund eines geringeren Energiebedarfs für die Zerkleinerung. Die erfindungsgemäßen Treibmittelpräpärationen weisen ferner vorzugsweise eine breitere bzw. universellere Anwendbarkeit, ein gutes Fließverhalten sowie eine gute Lagerfähigkeit auf.

Diese Aufgabe wurde gelöst mittels Treibmittelpräparationen enthaltend
a) wenigstens ein organisches und/oder ein anorganisches Treibmittel und
b) gegebenenfalls eine oberflächenaktive Verbindung,
wobei die Treibmittelpräparationen einen Wassergehalt von weniger als 3 Gew.-%, bezogen auf die Treibmittelpräpäration, bevorzugt weniger als 1 Gew.-% und eine mittlere Partikelgröße von 20 bis 5000 *µ*m, vorzugsweise von 100 bis 1000 *µ*m aufweisen. Der Ausdruck mittlere Partikelgröße bezieht sich in diesem Zusammenhang auf Partikel in Form von Granalien bzw. Agglomeraten, die im Allgemeinen aus einer Vielzahl von kleineren sog. Einzel- oder Primärpartikeln bestehen. Unter mittlere Partikelgröße sei im Rahmen dieser Anmeldung das Zahlenmittel bei Bestimmung mittels mikroskopischer Auszählung der Granalien oder der Medianwert der Masseverteilung bei Siebanalyse verstanden. Die erfindungsgemäße feste Treibmittelpräparation basiert vorzugsweise auf einer zylindrischen oder sphärischen Partikelstruktur, besonders bevorzugt auf einer kugelförmigen bis kugelformahnlichen Partikelstruktur, auf Agglomeraten kleinerer kugelformähnlicher Partikel und/oder Agglomeraten von Primärpartikeln.

Die Treibmittelpräparationen weisen vorzugsweise
a) 2 bis 99,99 Gew.-%, insbesondere 70 bis 99 Gew.-%, bezogen auf die gesamte Treibmittelpräparation wenigstens eines organischen und/oder anorganischen Treibmittels und
b) 0 bis 10 Gew.-%, vorzugsweise 0,01 bis 5 Gew.-%, bezogen auf die gesamte Treibmittelpräparation der oberflächenaktiven Verbindung und
c) ggf. bis zu 98 Gew.-%, bezogen auf die gesamte Treibmittelpräparation weitere Zuschlagsstoffe auf,
wobei die Summe der Gew.-% der organischen und/oder anorganischen Treibmittel, der oberflächenaktiven Verbindung und ggf. weiteren Zuschlagstoffen 100 Gew.-% ergeben muss.

Die organischen und/oder anorganischen Treibmittel werden ausgewählt aus den konventionell bekannten Treibmitteln und unterliegen erfindungsgemäß keinen Einschränkungen. Im Allgemeinen handelt es sich um feste, kristalline und/oder amorphe, organische oder anorganische, insbesondere nicht wasserlösliche Verbindungen.

Als organische Treibmittel werden vorzugsweise Azodicarbonamid (ADCA), Hydrazodicarbonamid (HDCA), Oxi-bis-sulfo-hydrazid (OBSH) (= p,p'-Oxy-bis(benzolsulfonsäurehydrazid), Toluol-sulfo-hydrazid (TSH) (= p-Toluolsulfonsäurehydrazid), Dinitropentamethylentetramin (DPT), 5-Phenyltetrazol (5 PT), Benzol-sulfo-hydrazid (BSH), (= Benzolsulfonylhydrazid), Paratoluol-sulfonyl-semicarbazid (PTSS), und deren Salze, insbesondere Alkali- und Erdalkalimetallsalze eingesetzt. Bevorzugt ist Azodicarbonamid.

Als anorganisches Treibmittel werden vorzugsweise Natriumhydrogencarbonat oder wasserfreies Mononatriumzitrat eingesetzt.

Die organischen und/oder anorganischen Treibmittel werden vorzugsweise alleine oder in Mischungen untereinander eingesetzt.

Die organischen und/oder anorganischen Treibmittel werden vorzugsweise als wässrige Synthesesuspension, trockenes Pulver, wasserfeuchte Filter-, Nutsch- und/oder als Presskuchen eingesetzt. Bevorzugt werden die organischen und/oder anorganischen Treibmittel gereinigt von synthesebedingte Nebenprodukten wie Salzen, Säure- und/oder Laugenresten eingesetzt.

Die organischen und/oder anorganischen Treibmittel besitzen vorzugsweise eine mittlere Primärpartikelgröße von 0,1 bis 100 *µ*m, bevorzugt 0,5 bis 50 *µ*m, besonders bevorzugt 1 bis 30 *µ*m. Unter mittlere Primärpartikelgröße sei im Rahmen dieser Anmeldung der Medianwert der Volumenverteilung der Primärpartikel (Einzelpartikel) verstanden, wie sie beispielsweise mittels Laserstreulichtverteilungsanalyse oder Lasergranulometrie (Laserbeugungsanalyse) ermittelt werden kann.

Erfindungsgemäß besteht bezüglich der gegebenenfalls zu verwendenden oberflächenaktiven Verbindungen keine Einschränkung, jedoch werden als oberflächenaktive Verbindungen vorzugsweise in Wasser vollständig oder teilweise lösliche bzw. emulgierbare Emulgatoren, Netzmittel, Dispergiermittel, Entschäumer oder Lösungsvermittler verstanden. Insbesondere können sie nichtionogen, anionogen, katiogen oder amphoter sein bzw. monomerer, oligomerer oder polymerer Natur sein.

Die oberflächenaktiven Verbindungen sind vorzugsweise Netzmittel und/oder Dispergiermittel, die in Wasser bei Raumtemperatur eine Löslichkeit von mehr als 0,01 g/l, vorzugsweise mehr als 0,1 g/l aufweisen, und die in organischen Medien eine Löslichkeit von mehr als 20 Gew.-%, vorzugsweise mehr als 40 Gew.-% bezogen auf die gesamte Lösung aufweisen. Unter organische Medien seien im Sinne der Erfindung polare und unpolare Lösungsmittel, Kohlenwasserstoffe, Öle, Fette und insbesondere Polymere verstanden.

Die oberflächenaktiven Verbindungen werden vorzugsweise aus der Gruppe der Alkoxylate, Alkylolamide, Ester, Aminoxide und/oder Alkylpolyglykoside ausgewählt.

Besonders bevorzugt werden die oberflächenaktiven Verbindungen ausgewählt aus der Gruppe der Umsetzungsprodukte von Alkylenoxiden mit alkylierbaren Verbindungen, insbesondere Alkylenoxidaddukte aus der Klasse der Umsetzungsprodukte von Ethylenoxid und/oder Propylenoxid mit
- gesättigten und/oder ungesättigten Fettalkoholen mit 6 bis 25 C-Atomen;
- Alkylphenolen mit 4 bis 12 C-Atomen im Alkylrest;
- gesättigten und/oder ungesättigten Fettaminen mit 14 bis 20 C-Atomen;
- gesättigten und/oder ungesättigten Fettsäuren mit 14 bis 22 C-Atomen;
- hydrierten und/oder unhydrierten Harzsäuren;
- aus natürlichen oder modifizierten, ggf. hydrierten Rizinusölfettkörper hergestellte Veresterungs- und/oder Arylierungsprodukte, die ggf. durch Veresterung mit Dicarbonsäuren zu wiederkehrenden Struktureinheiten verknüpft sind.

Die oberflächenaktiven Verbindungen werden vorzugsweise aus der Gruppe der
- Sorbitanester (SPAN®, ICI);
- die Umsetzungsprodukte von Alkylenoxid mit Sorbitanester (Tween®, ICI);
- Block- und Blockcopolymere auf Basis von Ethylen- und/oder Propylenoxid (Pluronic®, BASF);
- Block- und Blockcopolymere von Ethylen- und/oder Propylenoxid auf bifunktionellen Aminen (Tetronic®, BASF);
- Blockcopolymere auf Basis von (Poly)stearinsäure und (Poly)Alkylenoxid (Hypermer® B, ICI), oxalkylierte Acetylendiole und -glykole (Surfynol®, AirProducts);
- Polymere, aufgebaut aus wiederkehrenden Succinyl-Einheiten, insbesondere Polyasparaginsäure;
- ionische oder nichtionische polymere oberflächenaktive Verbindungen aus der Gruppe der Homo- und Copolymerisate, Pfropf- und Pfropfcopolymerisate sowie statistische und lineare Blockcopolymerisat wie beispielsweise Polyethylenoxide, Polypropylenoxide, Polyoxymethylene, Polytrimethylenoxide, Polyvinylmethylether, Polyethylenimine, Polyacrylsäuren, Polyarylamide, Polymethacrylsäuren, Polymethacrylamide, Poly-N,N-dimethyl-acrylamide, Poly-N-isopropylacrylamide, Poly-N-acrylglycinamide, Poly-N-methacrylglycinamide, Polyvinyloxazolidone, Polyvinylmethyloxazolidone;
- anionische oberflächenaktive Verbindungen wie bspw. Alkylsulfate, Ethersulfate, Ethercarboxylate, Phosphatester, Sulfosuccinatamide, Paraffinsulfonate, Olefinsulfonate, Sarcosinate, Isothionate und Taurate; oder
- amphotere oberflächenaktive Mittel wie Betaine und Ampholyte, insbesondere Glycinate, Propionate und Imidazoline,
ausgewählt.

Die oberflächenaktive Verbindung weist vorzugsweise einen ionisch modifizierten Phenol/Styrol-Polyglykolether auf. Unter ionische Modifizierung wird beispielsweise Sulfatierung, Carboxylierung oder Phosphatierung verstanden. Ionisch modifizierte Verbindungen liegen vorzugsweise als Salz, insbesondere als Alkali- oder Aminsalz, vorzugsweise Diethylaminsalz vor. Bevorzugt werden oberflächenaktive Verbindungen aus der Gruppe der oxalkylierte Phenole mit der Formel I) oder II) 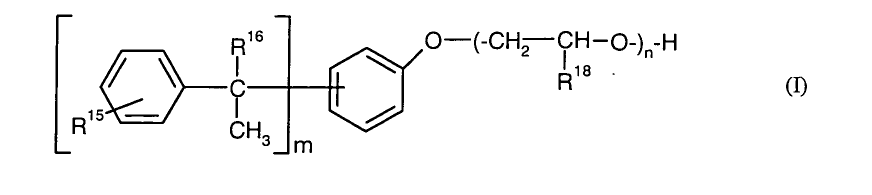 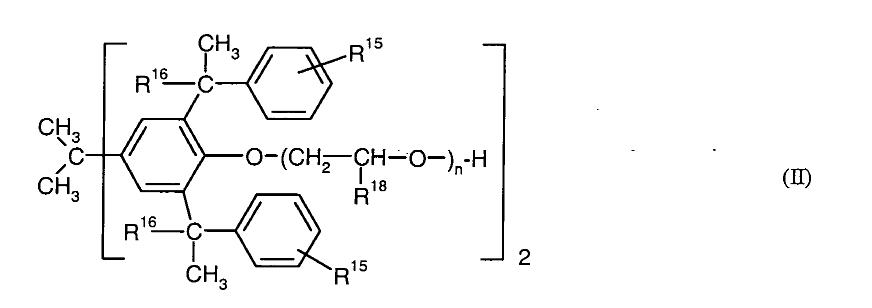 ausgewählt, in denen
- R¹⁵: für H oder C₁-C₄-Alkyl steht,
- R¹⁶: für H oder CH₃ steht,
- R¹⁷: für H, C₁-C₄-Alkyl, C₁-C₄-Alkoxy, C₁-C₄-Alkoxycarbonyl oder Phenyl steht,
- m: für eine Zahl von 1-4 steht,
- n: für eine Zahl von 2 bis 50, vorzugsweise von 2 bis 16 steht,
- R¹⁸: für jede durch n indizierte Einheit gleich oder verschieden ist und für Wasserstoff, CH₃ oder Phenyl steht, wobei im Falle der Mitanwesenheit von CH₃ in den verschiedenen -(-CH₂-CH(R¹⁸)-O-)-Gruppen in 0 bis 60 % des Gesamtwertes von n R¹⁸ für CH₃ und in 100 bis 40 % des Gesamtwertes von n R¹⁸ für Wasserstoff steht und wobei im Falle der Mitanwesenheit von Phenyl in den verschiedenen -(-CH₂-CH(R¹⁸)-O-)-Gruppen in 0 bis 40 % des Gesamtwertes von n R¹⁸ für Phenyl und in 100 bis 60 % des Gesamtwertes von n R¹⁸ für Wasserstoff steht.
die gegebenenfalls ionisch modifiziert sind.

Die oberflächenaktiven Verbindungen werden vorzugsweise ausgewählt aus der Gruppe der Dispergiermittel, insbesondere der Kondensationsprodukte, die durch Umsetzung von Naphtholen mit Alkanolen, Anlagerung von Alkylenoxid und mindestens teilweiser Überführung der terminalen Hydroxygruppen in Sulfogruppen oder Halbester der Maleinsäure, Phthalsäure oder Bernsteinsäure erhältlich sind, der Alkylarylsulfonate wie Alkylbenzol- oder Alkynaphthalensulfonate, sowie der Salze der Polyacrylsäuren, Polyethylensulfonsäuren, Polystyrolsulfonsäure, Polymethacrylsäuren, Polyphosphorsäuren.

Bevorzugt werden insbesondere Alkylbenzolsulfonate der Formel III 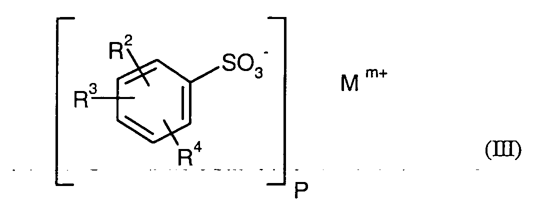 in der
- R², R³, R⁴: für H oder einen C₁ -C₂₄-Alkylrest stehen,
wobei mindestens einer der Substituenten R², R³, R⁴ ungleich Wasserstoff ist,
- p: für 1 oder 2 steht,
- M: für H, einen Ammonium-Rest oder ein Alkalimetall steht, wenn m = 1, und für ein Erdalkalimetall, wenn m = 2, und
R², R³, R⁴ für H, und einen C₆-C₁₈-Alkylrest steht.

Die oberflächenaktiven Verbindungen können vorzugsweise aus der Gruppe der Sulfobernsteinsäuremono- und -diester und ihrer Salze ausgewählt werden, insbesondere die der Formel IV 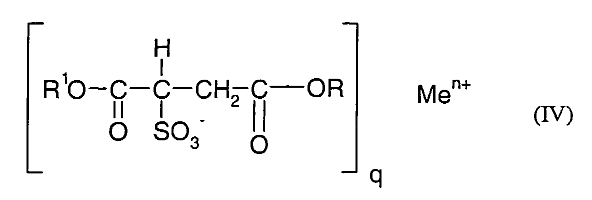 aufweisen, in der
- R, R¹: für H oder einen C₁-C₂₄₋ Kohlenstoffrest stehen, vorzugsweise für einen C₆-C₁₈- Alkyl- oder Aralkylrest, wobei R und R¹ jedoch nicht gleichzeitig H bedeuten,
- q: für 1 oder 2 steht und
- Me: für H, einen Ammonium-Rest oder ein Alkalimetall wenn n = 1, und für ein Erdalkalimetall wenn n = 2 steht.

Besonders bevorzugte oberflächenaktive Verbindungen sind Block- und Blockcopolymere auf Basis von Ethylen- und/oder Propylenoxid, ggf. ionisch modifizierte Phenol/Styrol-Polyglykolether der Formel I) und II), Alkylbenzolsulfonate der Formel III) sowie Diester der Sulfobernsteinsäure und ihre Salze gemäß Formel IV). Ganz besonders bevorzugt ist Natriumbistridecylsulfosuccinat, Natriumdioctylsulfosuccinat, Natriumdihexylsulfosuccinat, Natriumdiamylsulfosuccinat sowie Mischungen davon.

Erfindungsgemäß können vorzugsweise auch Mischungen der oberflächenaktiven Verbindungen eingesetzt werden.

Bevorzugte Treibmittelpräparationen enthalten als organisches und/oder anorganisches Treibmittel Azodicarbonamid und gegebenenfalls eine oberflächenaktive Verbindung aus Natriumbistridecylsulfosuccinat, Natriumdioctylsulfosuccinat, und/oder Natriumdihexylsulfosuccinat, Natriumdiamylsulfosuccinat. Diese Treibmittelpräparationen können ferner Wasserabsorbentien wie Silicagel, Zeolite, Aluminiumoxid, Magnesiumoxid, Magnesiumhydroxid, Calziumoxid, Calziumhydroxid, organische Säureanhydride und/oder wasserfreie anorganische Salze, insbesondere Magnesiumsulfat und/oder Natriumcarbonat, als Zuschlagstoff enthalten.

Andere bevorzugte Treibmittelpräparationen enthalten als organisches und/oder anorganisches Treibmittel Azodicarbonamid und gegebenenfalls als oberflächenaktive Verbindung Block- und Blockcopolymere auf Basis von Ethylen- und/oder Propylenoxid.

Noch andere Treibmittelpräparationen enthalten vorzugsweise als organisches und/oder gegebenenfalls anorganisches Treibmittel Azodicarbonamid und als oberflächenaktive Verbindung ein Alkylbenzolsulfonat der Formel III.

Die Treibmittelpräparationen enthalten vorzugsweise weitere Zuschlagstoffe. Als Zuschlagstoffe werden vorzugsweise Stabilisatoren, Farbmittel wie z.B. Dispersionsfarbstoffe, Pigmente und/oder Füllstoffe, Schauminhibitoren, Haftvermittler (sog. coupling agents), Wasserabsorbentien und/oder organische Lösungsmittel oder Mischungen davon verwendet.

Als Stabilisatoren werden vorzugsweise tribasisches Bleisulfat, dibasische Phosphite, Bleistearat, Zinkstearat, Zinkcarbonat, Zinkoxid, Bariumstearat, Aluminiumstearat, Calciumstearat, Dibutyltinmaleat, und/oder Harnstoff verwendet. Besonders bevorzugt werden PVC-Stabilisatoren.

Als Farbmittel werden vorzugsweise organo-chemische Verbindungen, die einen Schmelzpunkt von > 40°C und eine Löslichkeit in Wasser bei 20°C von < 10 g/l, insbesondere < 1 g/l besitzen, verwendet. Bevorzugt sind Dispersionsfarbstoffe oder Solventfarbstoffe zu nennen, wie sie z.B. im Colour Index, 3. - Auflage (3. Revision 1987) unter "Disperse Dyes" beschrieben sind, bzw. in Colour Index, 3. Auflage (1982, Pigments and Solvent Dyes) beschrieben sind.

Als Dispersionsfarbstoffe werden vorzugsweise carbonsäure- und/oder sulfosäuregruppenfreie Nitro-, Amino-, Aminoketon, Ketoninim, Methin-, Polymethin-, Diphenylamin-, Chinolin-, Benzimidazol, Xanthen-, Oxazin-, Cumarin, bevorzugt Anthrachinon- und Azofarbstoffe, wie Mono- und Disazofarbstoffe, verwendet. Besonders bevorzugt sind Dispersionsfarbstoffe wie sie in den Formeln 1) bis 23) aus EP 924335 A1 entnommen werden können.

Als Pigmente und/oder Füllstoffe kommen vorzugsweise alle nach dem Stand der Technik bekannten infrage wie z.B. zu entnehmen aus: Lückert, Pigment + Füllstoff Tabellen, 5. Auflage, Laatzen, 1994. Hierbei handelt es sich insbesondere um in wässrigen Medien unlösliche Stoffe.

Als Pigmente und/oder Füllstoffe werden vorzugsweise anorganische Weißpigmente, wie Titandioxid, Zinkoxid (wie ZnO, Zinkweiß), Zirkonoxid, Carbonate, Sulfate, Sulfide und Lithopone, insbesondere Titandioxid, verwendet.

Als Pigmente und/oder Füllstoffe werden vorzugsweise auch anorganische Buntpigmente aus der Gruppe der Oxide und Hydroxide in Form ihrer anorganischen Einzelverbindungen oder Mischphasen, insbesondere Eisenoxidpigmente, Chromoxidpigmente und oxidische Mischphasenpigmente mit Rutil- oder Spinellstruktur, Bismutvanadat-, Cadmium-, Cersulfid-, Chromat-, Ultramarin- und Eisenblaupigmente, verwendet.

Als Pigmente und/oder Füllstoffe werden vorzugsweise Eisenoxidpigmenten wie im Color Index Pigment Yellow 42, Pigment Red 101, Pigment Blue 11, Pigment Brown 6 sowie transparente Eisenoxidpigmente verwendet. Bevorzugt werden Chromoxidpigmenten vom Color Index Pigment Green 17 und Pigment Green 18. Bevorzugte Beispiele von oxidischer Mischphasenpigmente sind Nickeltitan- und Chromtitangelb, Cobaltgrün und -blau, Zinkeisen- und Chromeisenbraun sowie Eisenmangan- und Spinellschwarz. Bevorzugt sind auch Eisenoxidpigmente, dabei sind rote Eisenoxidpigmente besonders bevorzugt.

Als Pigmente und/oder Füllstoffe können vorzugsweise organische Pigmente wie solche der Monoazo-, Disazo-, verlackte Azo-, β-Naphthol-, Napthol AS-, Benzimidazolon-, Disazokondensations-, Azo-metallkomplex-, Isoindolin- und Isoindolinon-Reihe, sowie polycyclische Pigmente wie z.B. aus der Phthalocyanin-, Chinacridon-, Perylen-, Perinon-, Thioindigo-, Anthrachinon-, Dioxazin Chinophthalon- und Diketopyrrolopyrrol-Reihe verwendet werden. Außerdem können vorzugsweise verlackte Farbstoffe wie Ca-, Mg- und Al-Lacke von sulfonsäure- oder carbonsäuregruppenhaltigen Farbstoffen, sowie auch Ruße, die im Rahmen dieser Anmeldung als Pigmente verstanden werden und von denen eine große Zahl beispielsweise aus Colour Index, 2. Auflage [Verlag, Jahr], bekannt sind, verwendet werden. Bevorzugt sind saure bis alkalische Ruße die nach dem Furnacerußverfahren hergestellt werden sowie chemisch oder flächenmodifizierte Ruße wie sulfo- oder carboxylgruppenhaltige Ruße.

Als Pigmente und/oder Füllstoffe können vorzugsweise auch anorganische Füllstoffe wie Calciumcarbonat, Talkum, Glimmer, und/oder Bariumsulfat verwendet werden. Bevorzugt sind hydrophobierte hochdisperse, amorphe pyrogene Kieselsäuren, feinstteiliges, hydrophobiertes Kaolin und/oder hochdisperses Aluminiumoxid.

Als Schauminhibitoren werden vorzugsweise Maleinsäuren verwendet.

Als Haftvermittler (coupling agents) werden vorzugsweise Silane-, Aluminium- und Titanatcoupling agents verwendet. Weitere bevorzugte Haftvermittler sind in EP-A 0 943 655 näher beschrieben.

Als Wasserabsorbentien werden vorzugsweise Silicagel, Zeolite, Aluminiumoxid, Magnesiumoxid, Calziumoxid, organische Säureanhydride und/oder wasserfreie anorganische Salze, insbesondere Magnesiumsulfat und/oder Natriumcarbonat, verwendet.

Weitere bevorzugte Zuschlagstoffe sind Magnesium- oder Calziumhydroxid.

Die organischen Lösungsmittel sind vorzugsweise wasserlöslich bzw. wassermischbar oder wasserunlöslich. Als wasserunlösliche organische Lösungsmittel werden vorzugsweise die mit einem Schmelzpunkt von weniger als 90°C, insbesondere bei Raumtemperatur flüssige Lösungsmittel aus der Gruppe der aliphatischen, cycloaliphatischen oder aromatischen Kohlenwasserstoffe, insbesondere Mineralöle, Paraffine, Isoparaffine, vollsynthetische Öle, halbsynthetische Öle, mittlerer und ungesättigter Fettsäuren, etherische Öle, gereinigte natürliche Öle und Fette, Ester von natürlichen oder synthetischen, gesättigten oder ungesättigten Fettsäuren, C₈-C₂₂-Fettsäuren, alkylierte Aromaten und deren Gemische (z.B. Solvesso®), alkylierte Alkohole und/oder durch Hydroformylierung gewonnene lineare, primäre Alkohole (z.B. Dobanole®), verwendet.

Die wassermischbaren oder wasserlöslichen organischen Lösungsmittel besitzen vorzugsweise einen Siedepunkt oberhalb von 150°C, insbesondere oberhalb von 250°C. Unter wasserlöslich sei verstanden, dass die Verbindungen eine Löslichkeit in Wasser bei Raumtemperatur von >1 g/l, insbesondere >5 g/l aufweisen. Unter wassermischbar sei verstanden, dass sich die Verbindungen in einer Konzentration von >5 g/l, insbesondere >10 g/l bei Raumtemperatur aus Wasser nicht entmischen.

Bevorzugte organische Lösungsmittel sind Polyglykole oder Diole mit mindestens einer endständigen von Wasserstoff verschiedenen Gruppe, insbesondere Verbindungen aus den Gruppen der
- Ethylenglykolmonoalkylether, insbesondere -methylether (Methyltetraglykol) und/oder -butylether (Butyldiglykol),
- Ethylenglykoldialkylether, insbesondere Tri- und Tetraethylenglykoldimethylether,
- Propylenglykoldimethylether,
- Polyethylenglykoldimethylether
- Polyethylenglykoldibutylether, insbesondere mit 2 bis 5 Moleinheiten Ethylenoxid,
- Polyethylenglykolmonoallylether, -diallylether und -allylmethylether.

Besonders bevorzugt sind Tetraethylenglykoldimethylether sowie Polyethylenglykoldimethylether mit 3 bis 22, vorzugsweise 3 bis 12 Moleinheiten Ethylenglykol.

Die Erfindung betrifft auch ein Verfahren zur Herstellung der oben beschriebenen Treibmittelpräparationen, dadurch gekennzeichnet, dass
1) wenigstens ein organisches und/oder ein anorganisches Treibmittel, zusammen mit gegebenenfalls einer oberflächenaktiven Verbindung und/oder gegebenenfalls Zuschlagstoffen in Wasser eingebracht wird und zu einer Suspension homogenisiert wird,
2) die Suspension aus Schritt 1) ggf. auf eine mittlere Primärpartikelgröße des Treibmittels von 0,1 bis 100 *µ*m, bevorzugt 0,5 - 50 *µ*m besonders bevorzugt 1 - 30 *µ*m nasszerkleinert wird,
3) die nasszerkleinerte Suspension aus Schritt 2) getrocknet wird, und
4) das getrocknete Produkt aus Schritt 3) ggf. zum Granulat aufgearbeitet wird,
wobei Schritt 3) und 4) in umgekehrter Reihenfolge bzw. gleichzeitig stattfinden können, und der Gehalt an organischen und/oder anorganischen Treibmittel, ggf. der oberflächenaktiven Verbindung und ggf. Zuschlagstoffe zusammen 1 bis 80 Gew.-%, vorzugsweise 30 bis 60 Gew.-%, bezogen auf die Suspension vor der Trocknung in Schritt 3) beträgt.

Die anorganischen und/oder organischen Treibmittel werden vorzugsweise in fester Form als gefinishte oder ungefinishte Pulver, wässrige Synthesesuspension oder als wasserfeuchte Filter-, Nutsch- oder Presskuchen in einem wässrigen Medium zusammen mit gegebenenfalls einem Teil der oberflächenaktiven Verbindung sowie gegebenenfalls weiteren Zuschlagstoffen kontinuierlich oder diskontinuierlich eingetragen, nasszerkleinert, ggf. eingedickt und/oder isoliert (filtriert) und anschließend granuliert und getrocknet oder direkt zum Granulat getrocknet.

Vorzugsweise wird ein wässriges Medium verwendet, das einen pH-Wert von 2 bis 12, vorzugsweise von 2 bis 10 aufweist; bevorzugt liegt der pH Wert während der Nasszerkleinerung oberhalb des pH Wertes des isoelektrischen Punktes des organischen und/oder anorganischen Treibmittels im Wasser. Im Allgemeinen wird eine Temperatur von 0 bis 95°C, vorzugsweise von 20 bis 60°C zur kontinuierlichen oder diskontinuierlichen Nasszerkleinerung angelegt.

Die Nasszerkleinerung in Schritt 2) erfolgt vorzugsweise mittels Schnellrührer, Dissolver, UltraTurrax, Rotor-Stator-Mühlen, Inline-Mischer, langsamlaufenden Rührwerkskugelmühlen oder Zentrifugalmühlen mit Energiedichten von 0,1 bis 0,5 kW/l, bezogen auf den wirksamen Mahlraum, oder mittels schnelllaufenden Rührwerkskugel- und -perlmühlen mit einer Energiedichte von 0,5 bis 3 kW/1. Ferner kommen Dispersionskneter, Walzenstuhl oder Hochdruckhomogenisator als Mahlaggragate in Betracht.

Unter Nasszerkleinerung wird in dem Zusammenhang Homogenisieren (=Desagglomerieren), Mahlen zur Primärpartikelzerkleinerung sowie auch Kneten in wässriger Suspension verstanden. Dieser Verfahrensschritt überführt die im Allgemeinen synthesebedingt grobteiligen Primärpartikel der Treibmittel in den gewünschten feinverteilten Zustand. Die ggf. erforderlichen oberflächenaktiven Verbindungen sowie ggf. Zuschlagstoffe können gegebenenfalls vor, während oder nach der Erstellung der Feinverteilung zugesetzt werden. Die Auswahl der Verfahren zur Erzielung der gewünschten Feinverteilung vor der Trocknung richtet sich nach dem Aggregations- bzw. Agglomerationszustand der eingesetzten organischen und/oder anorganischen Treibmittel und dem erforderlichen Energieaufwand für Primärpartikelzerkleinerung selbst zur Erzielung der gewünschten Feinverteilung (Feinheitsgrade). Beispielsweise sind für Azodicarbonamid unterschiedliche Primärpartikelfeinheitsgrade von 30 *µ*m bis 2 *µ*m je nach Anwendungsgebiet erforderlich. Liegen die organischen und/oder anorganischen Treibmittel bei Eintrag bereits in der gewünschten Primärpartikelverteilung vor, so ist im Allgemeinen eine Homogenisierung ausreichend, sodass Verfahren wie Schnellrührer, Dissolver, Ultraturrax oder Rotor-Stator-Mühlen, sowie Inline-Mischer in Frage kommen. Wenn jedoch eine Primärpartikelzerkleinerung (Echtzerkleinerung) für die gewünschte Feinverteilung erforderlich ist, insbesondere bei hohen Feinheitsgraden (<=15*µ*m), können darüber hinaus Nassmahltechniken mit hohem bis sehr hohem Energieeintrag erforderlich sein. Dieser wird beispielsweise entweder durch langsam laufende Rührwerkskugelmühlen oder Zentrifugalmühlen mit einer Energiedichte von 0,1 bis 0,5 kW/l bezogen auf den wirksamen Mahlraum bereitgestellt, oder durch schnelllaufende Rührwerkskugel-und -perlmühlen mit einer Energiedichte von 0,5 bis 3 kW/l.

Vorzugsweise werden sog. Zentrifugalmühlen, beispielsweise Zentrifugalrohrmühlen (siehe z.B. Kurrer et al. TU Clausthal, "Zentrifugalrohrmühle zur Feinstzerkleinerung", Chemie Technik, 32. Jahrgang, 3/2003) sowie sog. Hochleistungs-Rührwerkskugelmühlen in vertikaler oder horizontaler Bauweise, bspw. vom Typ Advantis® Fa. Drais/Bühler AG, eingesetzt. Als Mahlperlen werden Metall- Glas- oder Keramikperlen eingesetzt, vorzugsweise Keramikperlen mit einem Durchmesser von 0,1 bis 5 mm, insbesondere 0,4 bis 2 mm verwendet.

Die Nasszerkleinerung in Schritt 2) findet vorzugsweise entweder diskontinuierlich oder kontinuierlich in Passagen- oder Kreislauffahrweise über eine oder mehrere Mahlaggregate mit ggf. unterschiedlichen Mahlkörpern statt.

Bei Kreislauffahrweise (Fig. 1) wird über Pumpe 2 und mindestens einem Mahlaggregat 4 ein Mahlkreislauf 3 mit hohem Mengenstrom erzeugt, dem die Treibmittelrohsuspension 1 kontinuierlich zugeführt und die erhaltene feinverteilte Treibmittelsuspension 5 in gleicher Menge kontinuierlich abgeführt wird.

Bei Passagenfahrweise (Fig. 2) sind mehrere Mahlaggregate 4, 4a in Reihe geschaltet, die Mahlsuspension wird mittels Pumpen 2, 2a kontinuierlich den Mahlaggregaten zugeführt.

Die so erhaltenen wässrigen Treibmittelsuspensionen werden gegebenenfalls anschließend mit ggf. weiteren organischen und/oder anorganischen Treibmitteln und/oder weiteren oberflächenaktiven Verbindungen und/oder weiterem Wasser und/oder weiteren genannten Zuschlagstoffen auf eine für die anschließende Trocknung gewünschte Zusammensetzung und Konsistenz eingestellt.

Erfindungsgemäß kann das organische und/oder anorganische Treibmittel auch in Form seiner wässrigen Synthesesuspension der Nassmahlung zugeführt werden, sodass ein zwischengestellter Isolierungsschritt entfällt. Nach der Nasszerkleinerung in Schritt 2) und vor der Trocknung in Schritt 3) findet ggf. eine Neutralisation und/oder einer Entfernung von synthesebedingten Nebenprodukten und/oder Salzen statt. Bevorzugt erfolgt dies mittels bekannten diskontinuierlichen Verfahren der Isolierung/Filtration auf z.B. Rührwerknutschen, Filterpressen, etc. Besonders bevorzugt sind jedoch kontinuierliche membrantechnischen Verfahren wie Micro- oder Ultrafiltration, insbesondere kontinuierliche Crossflow Microfiltration (z.B. Dynofilter®, Fa. Bokela) ggf. in Kombination mit Diafiltration.

Im Anschluss an die Nasszerkleinerung wird die wässrige Suspension vorzugsweise mittels Trocknung in die erfindungsgemäße feste Treibmittelpräparation überführt. Je nach Verfahren kann die Trocknung in Schritt 3) im Anschluss oder in Kombination mit einer Granulierung in Schritt 4) durchgeführt werden, ggf. kann eine Eindickung und/oder Isolierung (Filtrierung) der Mahlsuspension vor Trocknung/Granulierung zur Entfernung von überschüssigem Wasser erforderlich sein. Hinsichtlich der Kombination der genannten Verfahrensschritte besteht erfindungsgemäß keine Einschränkung, insbesondere werden die Trocknung und Granulation als erfmdungsgemäß zusammenhängende Verfahrensteile verstanden.

Die Trocknung in Schritt 3) bzw. die Granulierung nach Schritt 4) erfolgt vorzugsweise durch Sprühtrocknung, vorzugsweise Einstoffsprühtrocknung mittels Hochdruck- bzw. Drallkammerdüsen oder Sprühtrocknung mittels Zerstäubungsscheiben, Gefriertrocknung mit vor- oder nachgeschalteter Granulation oder Trockenaufarbeitung, Aufbaugranulation beispielsweise nach dem Teller- oder Trommelgranulationsverfahren gegebenenfalls mit teilweise vorgetrocknetem und/oder entfeuchtetem Produkt, Wirbelschichttrocknung und -granulation, sowie Mischeragglomeration und -trocknung gegebenenfalls in Kombination mit Wirbelschicht- bzw. Fließbetttrocknung. Ferner kommen vorzugsweise Verfahren wie Mischagglomeration in Suspension ggf. in Verbindung mit Brückenbildungsmitteln wie z.B. organische Lösungsmittel und mit gegebenenfalls nachgeschalteter Filtration und/oder Wirbelschicht- oder Fließbetttrocknung, Granulation mittels Pastenverformung und nachgeschalteter Nachtrocknung und Zerkleinerung oder Pelletierung sowie Dampfstrahlagglomeration in Frage. Kombinationen der genannten Verfahren sind ebenfalls möglich.

Besonders bevorzugt werden die Verfahren der einstufigen Sprühtrocknung mittels Zentrifugal-oder Düsenzerstäuber, ganz besonderes bevorzugt Hochdruck- bzw. Drallkammerdüsen, die Sprühtrocknung mit integrierter oder nachgeschalteter Fließbett- bzw. Wirbelschicht-Agglomeration und/oder -Trocknung, die Aufbaugranulation nach dem Tellerverfahren sowie die Fließbett- bzw. Wirbelschichtgranulation und -trocknung.

Erfindungsgemäß können ggf. weitere Zuschlagstoffe wie z.B. Entstaubungsmittel entweder der Suspension vor Trocknung/Granulierung zugegeben werden oder während oder nach der Trocknung auf die feste Treibmittelpräparation mit allgemein bekannten Verfahren aufgebracht werden.

Mit dem beschriebenen Verfahren und den so hergestellten Treibmittelpräparationen werden gegenüber dem Stand der Technik erhebliche Vorteile erzielt, u.a.:
1) um bis zu 12fach geringerer spezifischer Energieaufwand für die Zerkleinerung der Treibmittel im Vergleich zu konventioneller Luftstrahlmahlung; für Azodicarbonamid beispielsweise lässt sich bei mittleren Primärpartikelgrößen von 4 *µ*m ein spezifischer Energieeintrag von weniger als 1000, insbesondere weniger als 500 kJ/kg, bei 2 *µ*m weniger als 2000, insbesondere weniger als 1000 kJ/kg erreichen;
2) es können deutlich feinere mittlere Primärpartikeldurchmesser, bspw. kleiner als 2 *µ*m, insbesondere kleiner als 1 *µ*m erreicht werden als konventionell mit Luftstrahlmahlung wirtschaftlich möglich;
3) es können - insbesondere bei Azodicarbonamid - engere Primärpartikelgrößenverteilungen erhalten werden;
4) homogene Mischungen unterschiedlicher Treibmittel und Verteilungsbreiten sind im Status der wässrigen Suspensionen vor Trocknung/Granulation technisch einfach zu erzielen (i.V. dazu sind physikalisch homogene Trockenmischungen technisch kaum zu erzielen und zudem oft mit weiterer unerwünschter Primärpartikelzerkleinerung verbunden);
5) die erfindungsgemäßen festen Treibmittelpräparationen haben aufgrund ihrer Granulatstruktur und ihrer Zusammensetzung ein wesentlich verbessertes Staub- und Staubexplosionsverhalten sowie eine höhere Schüttdichte als bekannte Treibmittelpulver. Die erfindungsgemäßen Treibmittelpräparationen sind vorzugsweise staubarm oder sogar staubfrei und besitzen insbesondere einen Staubfilterwert von über 3. Als Granulate lassen sich die erfindungsgemäßen Treibmittelpräparationen zudem mittels Nachentstaubung erforderlichenfalls leicht staubarm oder staubfrei einstellen. Der Staubfilterwert wird nach einer Methode bestimmt, wie sie beispielsweise in Berger-Schunn et al, Bestimmung des Staubverhaltens von Farbstoffen, Textilveredelung 24 (1989), 7/8, S. 277-280 beschrieben ist. Hierbei wird der auftretende Staub über Filter abgesaugt und die Menge des Filterbelages visuell bestimmt. Ein Filterwert von 1 bedeutet hohe Staubentwicklung, ein Filterwert von 5 bedeutet, dass ein Staubbelag auf dem Filter nicht erkennbar ist und es sich um ein sehr staubarmes Produkt handelt. Sehr gut entstaubte Feststoffpräparationen müssen heute nach dem Stand der Technik auch nach mehrwöchiger Kalt- und Warmlagerung mindestens noch einen Filterwert von 3 aufweisen.
6) die erfindungsgemäßen festen Treibmittelpräparationen besitzen aufgrund ihrer Granulatstruktur und ihrer Zusammensetzung eine gute Lagerfähigkeit, insbesondere weisen sie auch bei mehrwöchiger Lagerung (RT und 40°C) gutes Fließ- und Staubverhalten auf und verklumpen nicht.
7) aufgrund ihrer Zusammensetzung und der gleichmäßigen Belegung der Primärpartikel im Inneren der Granulate besitzen die Treibmittelpräparationen eine ausgezeichnete Redispergierfähigkeit in organischen Medien, insbesondere in polymeren Matrixen;
8) die Treibmittelpräparationen sind in allen bekannten Anwendungen für Treibmittel einsetzbar, insbesondere sind sie geeignet zur Verschäumung von PVC, Gummi, Polyolefinen wie Polyethylen oder Polypropylen sowie anderen thermoplastischen Polymeren.

Zusammenhängend mit der o.g. Erfindung wurde überraschend ein neues Verfahren zur Herstellung von Azodicarbonamid durch,
1) die Umsetzung einer wässrigen Semicarbazidlösung und/oder Hydrazinhydrat mit Harnstoff, gegebenenfalls nach Ammoniakabtrennung, zu Hydrazodicarbonamid und
2) die Oxidation von Hydrazodicarbonamid mit einem Oxidationsmittel, vorzugsweise Chlor oder Wasserstoffperoxid, zu Azodicarbonamid,
dadurch gekennzeichnet, dass eine oberflächenaktive Verbindung vor und/oder während Schritt 1) und/oder vor oder während Schritt 2) eingesetzt wird.

Bevorzugt wird 0,001 - 2 Gew.-%, besonders bevorzugt 0,01 - 0,5 Gew.-% der oberflächenaktiven Verbindung bezogen auf den gebildeten Hydrazodicarbonamid in Schritt 1) und/oder Azodicarbonamid in 2) eingesetzt. Bevorzugte oberflächenaktive Mittel sind Alkylbenzolsulfonate der Formel III) sowie Diester der Sulfobernsteinsäure und ihre Salze gemäß Formel IV), insbesondere Natriumdioctylsulfosuccinat.

Die neue Synthese umfasst vorzugsweise mehrere Stufen. Die Synthese von Azodicarbonamid ist allgemein bekannt und erfolgt beispielsweise durch Überführung von Semicarbazid über die Zwischenstufe Hydrazodicarbonamid in Azodicarbonamid wie bspw. in EP 0 516 853 A1 oder DE 25 48 592 A1 beschrieben. Hydrazodicarbonamid wird bspw. synthetisiert, in dem eine wässrige Semicarbazidlösung, die z.B. durch Umsetzung von Hydrazinhydrat mit Harnstoff erhalten wurde, nach Entfernung von Ammoniak mit 1 bis 1,2 Mol Harnstoff pro Mol eingesetztem Semicarbazid umgesetzt wird (DE 24 52 016 A1).

Die Umsetzung wird vorzugsweise bei einem pH Wert von 7 oder weniger, wenn eingestellt durch Zugabe einer Säure, z.B. Schwefel- oder Salzsäure, und bei einer Temperatur von 90 bis 105°C durchgeführt, obwohl die Umsetzung nicht darauf beschränkt ist und auch bei höherem pH Wert durchgeführt werden kann.

Vorzugsweise erfolgt die Synthese von Semicarbazid und anschließende Umsetzung zu Hydrazodicarbonamid in einem Verfahrensschritt, ohne Zwischenisolierung des Semicarbazid.

Azodicarbonamid wird ebenfalls auf bekannte Weise synthetisiert, bspw. indem man das obige Hydrazodicarbonamid, entweder in der Form der Reaktionsmischung oder in Form isolierter Kristalle, in einem wässrigen Medium mit einem Oxidationsmittel, z.B. Chlor oder Wasserstoffperoxid, bei einer Temperatur von 10 bis 50°C oxidiert.

Mit dem erfindungsgemäßen Verfahren sind erhebliche technische Vorteile verbunden. Man erhält eine Viskositätsabsenkung der Synthesesuspensionen, insbesondere in der Reaktionsendphase beider Synthesestufen, dadurch einen verbesserten Energieeintrag ggf. verbunden mit einer verbesserten Reaktionsausbeute bzw. einer Verkürzung der Reaktionszeit. Man erhält ferner eine morphologisch einheitlichere, ggf. im Mittel gröbere Primärpartikelverteilung der Synthesesuspension mit besseren Filtrations- und Wascheigenschaften sowohl in der ggf. erforderlichen Zwischenisolierung von Hydrazodicarbonamid als auch der Isolierung/Wäsche von Azodicarbonamid. Damit verbunden sind weitere Vorteile wie niedrigerer Filtrationswiderstand und ein besserer Wascheffekt des Azodicarbonamid im Hinblick auf Nebenprodukte, Salze und Säurereste.

Anhand der nachfolgenden Beispiele wird die Erfindung näher erläutert, ohne dass dadurch eine Einschränkung der Erfindung bewirkt werden soll.

### Beispiel 1

In einem Rührwerkskessel wurden
- 25 Teile: vollentsalztes Wasser vorgelegt, unter Rühren
- 0,227 Teile: Natriumdioctylsulfosuccinat (Aerosol® OT 75, Cytec, Wirkstoffgehalt ca. 75 Gew.%) schaumfrei eingetragen und vollständig gelöst, anschließend
- 25 Teile: Azodicarbonamid in Form seines wasserfeuchten Filterkuchens mit pH 6,8, und einer Restfeuchte von 31,8 Gew.% schaumfrei eingetragen und homogenisiert. Der Medianwert der Primärpartikelverteilung d₅₀ betrug 25,4 *µ*m gemessen in verdünnter Suspension mittels Cilas® Lasergranulometer Typ 715 E090 (Laserbeugung, Fa Quantachrome). Vergleichsweise ergab eine Messung nach Trocknung einer Suspensionsprobe mittels Laserstreulichtanalyse (Fa.SYMPATEC, Typ Helos, Sensor 207, Dispergiersystem Rodos 1042): d₅₀ = 22,5 *µ*m, d₁₀ = 7,4 *µ*m, d₉₀ = 41,6 *µ*m

Die so erhaltene Suspension wurde anschließend in einer Mahlpassage auf einer schnelllaufenden, geschlossenen Rührwerkskugelmühle vom Typ Advantis® V15, Fa. Drais/Bühler mit 1200 ml Mahlraumvolumen, 600 U/min, Zirkonoxid-Mahlperlen im Durchmesser von 1,1-1,3 mm, Perlenfüllgrad 70 %, Produktdurchsatz 195 kg/h und einer Mahlleistung von 1,4 kW in einer Passage mit einem spezifischen Energieeintrag von 26 kJ/kg bezogen auf die Mahlsuspension, bzw. 76 kJ/kg bezogen auf eingesetztes Azodicarbonamid nassgemahlen. Mittels Lasergranulometrie ergab sich ein Medianwert der Primärpartikelverteilung d₅₀ von 13,5 *µ*m.

Vergleichsweise ergab eine Messung nach Trocknung einer Suspensionsprobe mittels Laserstreulichtanalyse:
d₅₀ = 13,2 *µ*m, d₁₀ = 5,4 *µ*m, d₉₀ = 31,5 *µ*m.

Die so erhaltene, sehr gut fliessfähige Treibmittelsuspension mit einem Feststoffgehalt von ca. 34 Gew.% wurde auf einem einstufiger Zerstäubungstrockner (Wasserverdampfungsleistung 80 kg/h) mit einer Hochdruckdrallkammerdüse (Fa. Delavan, Typ SDX F, Bohrung 1,4 mm) ohne Feingutrückführung zum Granulat getrocknet unter folgenden Bedingungen:
Düsendruck: 19 bar
Düsendurchsatz: 60 kg/h
Lufteintrittstemperatur: 130 °C
Luftaustrittstemperatur: 60 °C

Man erhielt eine erfindungsgemäße feste Treibmittelpräparation in Form eines staubfreien und sehr gut rieselfähigen Granulates mit einer mittleren Partikelgröße (mikroskopisch ausgezählt) von ca. 140 *µ*m und folgender Zusammensetzung (ca.):
98,8 Gew.% Azodicarbonamid (Treibmittel der Komp.a)
1,0 Gew.% Natriumdioctylsulfosuccinat (Verbindung der Komp.b)
0,2 Gew.% Wasser (Restfeuchte)

Diese feste Treibmittelpräparation war staubarm und sehr gut lagerstabil und für die Herstellung von vernetzten und unvernetzten PE-Schäumen sowie PP-Schäumen bestens geeignet.

Zur Nachentstaubung wurde ein Teil der festen Treibmittelpräparation labormäßig auf einer Rollenbank mit 0,4 Gew.% Weißöl (Primolöl® 352, Fa. Exxon-Mobil) bezogen auf die Präparation homogen gemischt. Man erhielt eine nahezu staubfreie Präparation (siehe Tabelle: Werte in Klammern).

Zum Vergleich wurde ein Teil des oben genannten Azodicarbonamid-Filterkuchens konventionell in einem Stromtrockner getrocknet, anschließend mittels einer Spiralstrahlmühle (Luftstrahlmühle) und einem spezifischen Energieaufwand von mehr als 1100 kJ/kg zum Pulver gemahlen; die erhaltene Primärpartikelverteilung war deutlich breiter:
d₅₀ = 15,2 *µ*m, d₁₀ = 3,6 *µ*m, d₉₀ = 34,9 *µ*m

### Prüfung des Schäumungsverhaltens:

15 Teile der festen Treibmittelpräparation und des Vergleiches wurden jeweils mit 100 Teilen LDPE ("low density polyethylene", Schmelzindex 2,0) und 0,8 Teilen Dicumylperoxid gemischt und mit einer Walzentemperatur von ca. 115°C auf einem Laborwalzenstuhl geknetet. Man erhielt jeweils Platten von 5mm Dicke, die unter Druck von 120 kg/cm² bei einer Temperatur von 125°C 5 Minuten lang gepresst wurden. Die aus den Platten entnommenen Proben wurden in einem Heißluftofen mit 220 °C geschäumt. In beiden Fällen erhielt man Schaumproben mit feinen und gleichmäßigen Zellen, glatter Oberfläche und vergleichbarer Schaumbildungsrate.

### Prüfung des Staubverhaltens:

Die feste Treibmittelpräparation, dieselbe mit Nachentstaubung (Werte in Klammern) sowie der Vergleich wurden wie oben beschrieben hinsichtlich ihres Staubfilterwertes vergleichend geprüft. Die Staubfilterwerte sofort nach Herstellung sowie nach 4 Wochen Lagerung bei Raumtemperatur und 40°C ergaben:

| | Treibmittelpräparation | Vergleich |
|---|---|---|
| sofort | 3 (4) | 1 |
| Lagerung 25°C | 2 (4) | 1 |
| Lagerung 40°C | 2 (4) | 1 |

### Beispiel 2

In einem Rührwerkskessel wurden
- 25 Teile: vollentsalztes Wasser vorgelegt, unter Rühren
- 0,17 Teile: eines Ethylenoxid-Propylenoxid-Block-Copolymeren (Pluronic® PE10500, BASF AG) eingetragen und vollständig gelöst, anschließend
- 25 Teile: Azodicarbonamid in Form seines wasserfeuchten Filterkuchens wie in Beispiel 1 beschrieben schaumfrei eingetragen und homogenisiert.

Die so erhaltene Suspension wurde wie in Beispiel 1 beschrieben bei einem Mahldurchsatz von 210 kg/h mit spezifischem Energieeintrag von 24 kJ/kg bezogen auf die Mahlsuspension, bzw. 71 kJ/kg bezogen auf eingesetztes Azodicarbonamid nassgemahlen. Mittels Lasergranulometrie gemessen betrug der Medianwert der Partikelverteilung d₅₀ 15,0 *µ*m.

Die nach Trocknung einer Probe mittels Laserstreulichtanalyse gemessene Primärpartikelverteilung ergab
d₅₀ = 14,6 *µ*m, d₁₀ = 4,7 *µ*m, d₉₀ = 27 *µ*m.

Die so erhaltene, ebenfalls sehr gut fließfähige Treibmittelsuspension wurde wie in Beispiel 1 beschrieben zum Granulat getrocknet unter folgenden Bedingungen:
Düsendruck: 21 bar
Düsendurchsatz: 69 kg/h
Lufteintrittstemperatur: 130°C
Luftaustrittstemperatur: 61°C

Man erhielt eine erfindungsgemäße feste Treibmittelpräparation in Form eines staubarmen und sehr gut rieselfähigen Granulates mit einer mittleren Partikelgröße (mikroskopische Auszählung) von ca. 140 *µ*m und folgender Zusammensetzung (ca.):
98,9 Gew. % Azodicarbonamid (Treibmittel der Komp.a)
1,0 Gew.% EO/PO-Blockcopolymer (Verbindung der Komp.b)
0,1 Gew.% Wasser (Restfeuchte)

Diese feste Treibmittelpräparation war sehr gut lagerstabil und für die Herstellung von vernetzten und unvernetzten PE-Schäumen sowie PP-Schäumen bestens geeignet.

### Prüfung des Schäumungsverhaltens:

Mit dem in Beispiel 1 beschriebenen Vergleichstest erhielt man in beiden Fällen Schaumproben mit feinen und gleichmäßigen Zellen, glatter Oberfläche und vergleichbarer Schaumbildungsrate.

Zur Nachentstaubung wurde ein Teil der festen Treibmittelpräparation labormäßig auf einer Rollenbank mit 0,2 Gew.% Weißöl (Primolöl® 352, Fa. Exxon-Mobil) homogen gemischt. Man erhielt eine nahezu staubfreie Präparation (siehe Tabelle: Werte in Klammern).

### Prüfung des Staubverhaltens:

Die feste Treibmittelpräparation und dieselbe nach Nachentstaubung wurden wie in Beispiel 1 beschrieben hinsichtlich ihres Staubfilterwertes vergleichend geprüft. Die Staubfilterwerte sofort nach Herstellung sowie nach 4 Wochen Lagerung bei Raumtemperatur und 40°C ergaben:

| | Treibmittelpräparation | Vergleich aus Bsp. 1 |
|---|---|---|
| sofort | 4 (5) | 1 |
| Lagerung 25°C | 3 (4) | 1 |
| Lagerung 40°C | 3 (4) | 1 |

### Beispiel 3

In einem Rührwerkskessel wurden
- 25 Teile: vollentsalztes Wasser vorgelegt, unter Rühren
- 0,227 Teile: Natriumdioctylsulfosuccinat (Aerosol® OT 75, Fa.Cytec, Wirkstoffgehalt ca. 75 Gew.%) schaumfrei eingetragen und vollständig gelöst, anschließend
- 25 Teile: Azodicarbonamid gemäß Beispiel 1
schaumfrei eingetragen und homogenisiert.

Die so erhaltene Suspension wurde anschließend in 1 Mahlpassage wie in Beispiel 1 beschrieben gemahlen, jedoch mit einem Leistungseintrag von 1,54 kW bei einem Durchsatz von 190 kg/h und einer Drehzahl von 800 U/min; anschließend wurden weitere
- 0,227 Teile: Natriumdioctylsulfosuccinat (Aerosol® OT 75, Fa. Cytec) und
- 0,017 Teile: Weißöl (Primolöl® 352, Fa. Exxon-Mobil)
schaumfrei in die Mahlsuspension eingetragen und eine weitere Passage unter den gleichen Bedingungen gemahlen.

Der spezifische Energieeintrag betrug insgesamt ca. 58 kJ/kg bezogen auf eingesetztes Azodicarbonamid. Der Medianwert der Primärpartikelverteilung d₅₀ gemessen mittels Lasergranulometrie betrug 7,0 *µ*m.

Die so erhaltene, ebenfalls sehr gut fliessfähige Treibmittelsuspension wurde wie in Beispiel 1 beschrieben zum Granulat getrocknet unter folgenden Bedingungen:
Düsendruck: 16 bar
Düsendurchsatz: 56 kg/h
Lufteintrittstemperatur: 130°C
Luftaustrittstemperatur: 60°C

Man erhielt eine erfindungsgemäße feste Treibmittelpräparation in Form eines staubarmen und sehr gut rieselfähigen Granulates mit einer mittleren Partikelgröße (mikroskopisch ausgezählt) von ca. 150 *µ*m und folgender Zusammensetzung (ca.):
97,7 Gew.% Azodicarbonamid (Treibmittel der Komp.a)
2,0 Gew.% Natriumdioctylsulfosuccinat (Verbindung der Komp.b)
0,1 Gew.% Weißöl
0,2 Gew.% Wasser (Restfeuchte)

Diese feste Treibmittelpräparation war sehr gut lagerstabil und staubarm.

### Prüfung des Staubverhaltens:

Die feste Treibmittelpräparation wurde wie in Beispiel 1 beschrieben hinsichtlich ihres Staubfilterwertes vergleichend geprüft. Als Vergleich wurde Handelsware Porofor® ADC-S/C2 (Bayer Chemicals AG, d50 6,7 *µ*m) herangezogen. Die Staubfilterwerte sofort nach Herstellung sowie nach 4 Wochen Lagerung bei Raumtemperatur und 40°C ergaben:

| | Treibmittelpräparation | Vergleich |
|---|---|---|
| sofort | 4 | 1 |
| Lagerung 25°C | 4 | 1 |
| Lagerung 40°C | 3 | 1 |

Bild 3 und Bild 4 zeigen lichtmikroskopische Aufnahmen der in Beispiel 3 beschriebenen festen Treibmittelpräparation (Bild 3) und des Vergleiches (Bild 4). Die Medianwerte der Primärpartikel sind wie beschrieben in etwa gleich groß.

### Beispiel 4

In einem Rührwerkskessel wurden
- 25 Teile: vollentsalztes Wasser vorgelegt, unter Rühren
- 0,227 Teile: Natriumdioctylsulfosuccinat (Aerosol® OT 75, Fa.Cytec, Wirkstoffgehalt ca. 75 Gew.%) schaumfrei eingetragen und vollständig gelöst, anschließend
- 25 Teile: Azodicarbonamid gemäß Beispiel 1
schaumfrei eingetragen und homogenisiert.

Die so erhaltene Suspension wurde anschließend in 4 Mahlpassagen unter den gleichen Bedingungen wie in Beispiel 1 beschrieben, jedoch mit einer Mahlleistung von 1,54 kW bei 800 U/min und einem Durchsatz von 190 kg/h gemahlen; anschließend wurden weitere
- 0,227 Teile: Natriumdioctylsulfosuccinat (Aerosol® OT 75, Fa. Cytec) und
- 0,017 Teile Weißöl: (wie in Beispiel 3)
schaumfrei in die Mahlsuspension eingetragen und eine weitere Passage mit einer Mahlleistung von 3,06 kW bei einer Drehzahl von 1200 U/min und einem Durchsatz von 160 kg/h gemahlen.

Der insgesamt eingetragene spezifische Energiee betrug ca. 183 kJ/kg bezogen auf Azodicarbonamid. Mittels Lasergranulometrie ergab sich ein Medianwert der Partikelverteilung d₅₀ von 4,3 *µ*m. Die nach Trocknung einer Suspensionsprobe und vorsichtiger Desagglomeration mittels Laserstreulichtanalyse gemessene Primärpartikelverteilung ergab
d₅₀ = 3,8 *µ*m, d₁₀ = 1,0 *µ*m, d₉₀ = 7,5 *µ*m, jedoch waren noch wenige Agglomerate oberhalb von 50 *µ*m zu erkennen.

Die so erhaltene, ebenfalls sehr gut fließfähige Treibmittelsuspension wurde wie in Beispiel 1 beschrieben zum Granulat getrocknet unter folgenden Bedingungen:
Düsendruck: 25 bar
Düsendurchsatz: 63 kg/h
Lufteintrittstemperatur: 130°C
Luftaustrittstemperatur: 63 °C

Man erhielt eine erfindungsgemäße feste Treibmittelpräparation in Form eines staubfreien und sehr gut rieselfähigen Granulates mit einer mittleren Partikelgröße (mikroskopische Auszählung) von ca. 170 *µ*m und folgender Zusammensetzung (ca.):
97,7 Gew.% Azodicarbonamid (Treibmittel der Komp.a)
2,0 Gew.% Natriumdioctylsulfocuccinat (Verbindung der Komp.b)
0.1 Gew.% Weißöl
0,2 Gew.% Wasser (Restfeuchte)

Diese feste Treibmittelpräparation war sehr gut lagerstabil und für die Verschäumung von PVC bestens geeignet.

Zum Vergleich wurde ein Teil des oben genannten Azodicarbonamid-Filterkuchens konventionell in einem Stromtrockner getrocknet, anschließend mittels einer Spiralstrahlmühle und einem spezifischen Energieeintrag von mehr als 6000 kJ/kg gemahlen; die erhaltene Primärpartikelverteilung war deutlich breiter:
d₅₀ = 3,93 *µ*m, d₁₀ = 0,88 *µ*m, d₉₀ = 8,82 *µ*m

### Prüfung des Staubverhaltens:

Die feste Treibmittelpräparation wurde wie in Beispiel 1 beschrieben hinsichtlich ihres Staubfilterwertes vergleichend geprüft. Die Staubfilterwerte sofort nach Herstellung sowie nach 4 Wochen Lagerung bei Raumtemperatur und 40°C ergaben:

| | Treibmittelpräparation | Vergleich |
|---|---|---|
| sofort | 4 | 1 |
| Lagerung 25°C | 4 | 1 |
| Lagerung 40°C | 3 | 1 |

### Beispiel 5

Mit dem gleichen Verfahren wie in Beispiel 3 beschrieben, jedoch ohne Zusatz eines oberflächenaktiven Mittels und ohne Weißöl erhielt man eine erfindungsgemäße feste Treibmittelpräparation in Form eines sehr gut rieselfähigen Granulates, aber noch schlechtem Staubverhalten. Die erhaltene mittlere Primärpartikelgröße betrug 6,9 *µ*m.

Zur Nachentstaubung wurden 0,6 Gew.% Polyethylenglykoldimethylether mit einer mittleren Molmasse von 350 g/mol zugesetzt und man erhielt ein ebenso gut fließfähiges und nahezu staubfreies Produkt.

| | Treibmittelpräparation | Treibmittelpräparation nachentstaubt |
|---|---|---|
| sofort | 3 | 5 |
| Lagerung 25°C | 2 | 4 |
| Lagerung 40°C | 2 | 4 |

## Patentansprüche

1. Treibmittelpräparationen enthaltend
a) wenigstens ein organisches und/oder ein anorganisches Treibmittel und
b) gegebenenfalls eine oberflächenaktive Verbindung,
wobei die Treibmittelpräparationen einen Wassergehalt von weniger als 3 Gew.-%, bezogen auf die Treibmittelpräpäration, vorzugsweise weniger als 1 Gew.-% und eine mittlere Primärpartikelgröße von 20 bis 5000 *µ*m, vorzugsweise von 100 bis 1000 *µ*m aufweisen.

2. Treibmittelpräparation gemäß Anspruch 1, **dadurch gekennzeichnet, dass** die Treibmittelpräparationen
a) 2 bis 99,99 Gew.-%, insbesondere 70 bis 99 Gew.-% bezogen auf die gesamte Treibmittelpräparation wenigstens eines organischen und/oder anorganischen Treibmittels,
b) 0 bis 10 Gew.-%, vorzugsweise 0,01 bis 5 Gew.-% bezogen auf die gesamte Treibmittelpräparation einer oberflächenaktiven Verbindung und
c) ggf. bis zu 98 Gew.-% bezogen auf die gesamte Treibmittelpräparation weitere Zuschlagstoffe enthalten,
wobei die Summe der Anteile der Komponenten 100 Gew.-% ergeben muss.

3. Treibmittelpräparationen gemäß Anspruch 1, **dadurch gekennzeichnet, dass** das Treibmittel der Komp.a) Azodicarbonamid , Hydrazodicarbonamid, Oxi-bis-sulfo-hydrazid (OBSH), Toluolsulfohydrazid (TSH), Dinitropentamethylentetramin (DPT), 5-Phenyltetrazol (5 PT), Benzolsulfohydrazid (BSH), Paratoluolsulfonylsemicarbazid (PTSS), oder deren Salze, insbesondere Alkali- und Erdalkalimetallsalze ist und eine mittlere Primärpartikelgröße (Medianwert der Primärpartikelverteilung) von 0,1 bis 100 *µ*m, vorzugsweise von 1 bis 30 *µ*m aufweist.

4. Treibmittelpräparationen gemäß den Ansprüchen 1 bis 3, **dadurch gekennzeichnet, dass** die Treibmittel der Komp.a) alleine oder in Mischungen untereinander eingesetzt werden.

5. Treibmittelpräparationen gemäß Anspruch 1. **dadurch gekennzeichnet, dass** die oberflächenaktive Verbindung ein Netzmittel und/oder Dispergiermittel ist, das in Wasser bei Raumtemperatur eine Löslichkeit von mehr als 0,01 g/l, vorzugsweise mehr als 0,1 g/l aufweist, und das in organischen Medien eine Löslichkeit von mehr 20 Gew.-%, vorzugsweise mehr als 40 Gew.-% bezogen auf die gesamte Lösung ausweist.

6. Treibmittelpräparationen gemäß Anspruch 1, **dadurch gekennzeichnet, dass** die oberflächenaktive Verbindung aus der Gruppe der Umsetzungsprodukte von Alkylenoxiden mit alkylierbaren Verbindungen, insbesondere der Alkylenoxidaddukte aus der Klasse der Umsetzungsprodukte von Ethylenoxid und/oder Propylenoxid mit
• gesättigten und/oder ungesättigten Fettalkoholen mit 6 bis 25 C-Atomen,
• Alkylphenolen mit 4 bis 12 C-Atomen im Alkylrest,
• gesättigten und/oder ungesättigten Fettaminen mit 14 bis 20 C-Atomen,
• gesättigten und/oder ungesättigten Fettsäuren mit 14 bis 22 C-Atomen,
• hydrierten und/oder unhydrierten Harzsäuren und/oder
• aus natürlichen oder modifizierten, ggf. hydrierten Rizinusölfettkörper hergestellte Veresterungs- und/oder Arylierungsprodukte, die ggf. durch Veresterung mit Dicarbonsäuren zu wiederkehrenden Struktureinheiten verknüpft sind,
ausgewählt wird.

7. Treibmittelpräparationen gemäß Anspruch 1, **dadurch gekennzeichnet, dass** die oberflächenaktive Verbindung aus der Gruppe der oxalkylierte Phenole mit der Formel I) oder 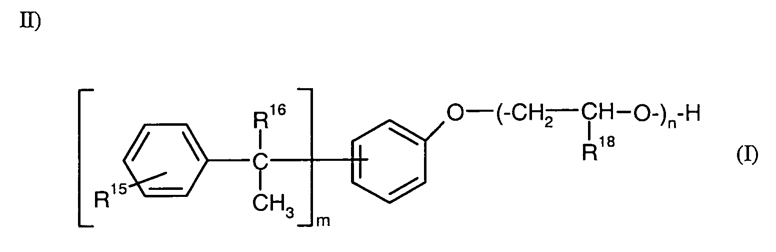 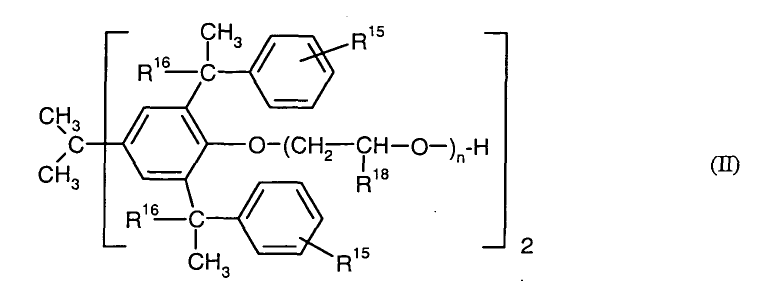 ausgewählt wird, in denen
R¹⁵ für H oder C₁-C₄-Alkyl steht,
R¹⁶ für H oder CH₃ steht,
R¹⁷ für H, C₁-C₄-Alkyl, C₁-C₄-Alkoxy, C₁-C₄-Alkoxycarbonyl oder Phenyl steht,
m für eine Zahl von 1-4 steht,
n für eine Zahl von 2 bis 50, vorzugsweise von 2 bis 16 steht,
R¹⁸ für jede durch n indizierte Einheit gleich oder verschieden ist und für Wasserstoff, CH₃ oder Phenyl steht, wobei im Falle der Mitanwesenheit von CH₃ in den verschiedenen -(-CH₂-CH(R¹⁸)-O-)-Gruppen in 0 bis 60 % des Gesamtwertes von n R¹⁸ für CH₃ und in 100 bis 40 % des Gesamtwertes von n R¹⁸ für Wasserstoff steht und wobei im Falle der Mitanwesenheit von Phenyl in den verschiedenen -(-CH₂-CH(R¹⁸)-O-)-Gruppen in 0 bis 40 % des Gesamtwertes von n R¹⁸ für Phenyl und in 100 bis 60 % des Gesamtwertes von n R¹⁸ für Wasserstoff steht,
die gegebenenfalls ionisch modifiziert sind.

8. Treibmittelpräparationen gemäß Anspruch 1, **dadurch gekennzeichnet, dass** die Treibmittelpräparationen Azodicarbonamid und gegebenenfalls eine oberflächenaktive Verbindung aus Block- und Blockcopolymere auf Basis von Ethylen- und/oder Propylenoxid enthalten.

9. Verfahren zur Herstellung der in den Ansprüchen 1 bis 8 beschriebenen Treibmittelpräparationen, **dadurch gekennzeichnet, dass**
1) wenigstens ein organisches und/oder ein anorganisches Treibmittel, zusammen mit gegebenenfalls einer oberflächenaktiven Verbindung und/oder gegebenenfalls Zuschlagstoffen in Wasser eingebracht wird und zu einer Suspension homogenisiert wird,
2) die Suspension aus Schritt 1) ggf. auf eine mittlere Primärpartikelgröße des Treibmittels von 0,1 bis 100 *µ*m, bevorzugt 0,5 - 50 *µ*m besonders bevorzugt 1 - 30 *µ*m nasszerkleinert wird,
3) die nasszerkleinerte Suspension aus Schritt 2) getrocknet wird, und
4) das getrocknete Produkt aus Schritt 3) ggf. zum Granulat aufgearbeitet wird,
wobei Schritt 3) und 4) in umgekehrter Reihenfolge bzw. gleichzeitig stattfinden können, und der Gehalt an organischen und/oder anorganischen Treibmittel, ggf. der oberflächenaktiven Verbindung und ggf. Zuschlagstoffe zusammen 1 bis 80 Gew.-%, vorzugsweise 30 bis 60 Gew.-%, bezogen auf die Suspension vor der Trocknung in Schritt 3) beträgt.

10. Verwendung der Treibmittelpräparationen gemäß Ansprüche 1 bis 8 als Treibmittel zur Verschäumung von thermoplastischen Polymeren.
